# FASCICULE DE BREVET EUROPEEN

(11) **EP 1 039 871 B1**
(45) Date de publication et mention de la délivrance du brevet: **10.03.2004**
(21) Numéro de dépôt: 98946206.4
(22) Date de dépôt: 12.10.1998
(51) Int. Cl.: A61H 9/00

(54) **APPAREIL INDUISANT UN ETAT DE RELAXATION PHYSIQUE ET MENTALE, PAR ECOULEMENT D'UN LIQUIDE TEMPERE SUR LE FRONT**
VORRICHTUNG ZUM INDUZIEREN KÖRPERLICHER UND GEISTIGER ENTSPANNUNG DURCH GIESSEN EINER FLÜSSIGKEIT AUF DIE STIRN
APPARATUS INDUCING PHYSICAL AND MENTAL RELAXATION, BY FLOW OF A TEMPERED LIQUID ON THE FOREHEAD

(30) Priorité: 20.10.1997 CH 243597
(43) Date de publication de la demande: 04.10.2000
(73) Titulaire: Martin, Bertrand, 1800 Vevey (CH)
(72) Inventeur: Martin, Bertrand, 1800 Vevey (CH)
(86) Numéro de dépôt international: PCT/CH1998/000436
(87) Numéro de publication internationale: WO 1999/020220

(56) Documents cités:
- DE-A- 2 508 000
- FR-A- 2 566 267
- US-A- 3 868 950

## Description

La présente invention concerne un appareil permettant l'écoulement sur le front d'une personne couchée d'un liquide tempéré, notamment de l'eau, induisant un profond état de relaxation physique et mentale.

Cette technique de la médicine ayurvédique indienne appelée Shirodhara est jusqu'à maintenant pratiquée par l'écoulement d'huile d'un pot percé suspendu au-dessus de la tête du patient. Ce pot doit être régulièrement rempli. La température de l'huile est difficile à maintenir constante. L'huile présente l'inconvénient, outre d'être d'un coût élevé, de tout enduire (tête, cheveux, table, matériel, etc) et l'hygiàne est difficile à maintenir. La main d'oeuvre est importante puisqu'une personne est nécessaire en permanence pour remplir le pot et balayer le front avec le jet d'huile. Ces manipulations provoquent en outre du bruit qui peut distraire le patient. Le dispositif actuel nécessite en outre une table de massage spéciale permettant de récupérer l'huile.

Les buts de l'invention sont de simplifier la technique décrite ci-dessus et de diminuer son coût. Ces buts sont atteints en utilisant un appareil selon l'invention défini à la revendication 1.

L'invention sera mieux comprise et ses avantages et ses caractéristiques apparaîtront plus clairement à la lecture de la description de formes d'exécution, donnée uniquement à titre d'exemple, en regard des dessins sur lesquels :
La figure 1 représente un premier exemple d'un appareil selon l'invention.
La figure 2 représente une variante.
La figure 3 représente une seconde variante.

Un appareil selon l'invention met en oeuvre une pompe 1, un chauffage à thermostat 2, un statif 3 et un support 4 pour la tête servant de collecteur de récupération du liquide, habituellement de l'eau, et un réservoir 5 pour stocké ledit liquide. Le liquide qui est maintenu à température constante par le chauffage à thermostat placé dans le réservoir 5 circule en circuit fermé. Du réservoir 5 le liquide est pompé dans un tube 6 supporté par un statif 3 et amené à une distance variable du front du patient sur lequel il s'écoule en flux continu, habituellement au centre du front. Si désiré, l'appareil peut comprendre un dispositif 7 permettant de déplacer le flux de liquide dans un va-et-vient latéral et continu afin d'opérer un balayage du front. L'appareil comprend une minuterie 8 permettant de programmer la durée du traitement L'appareil peut aussi comprendre un dispositif de sécurité 9 branché sur l'alimentation permettant de le déconnecter en de mauvais fonctionnement

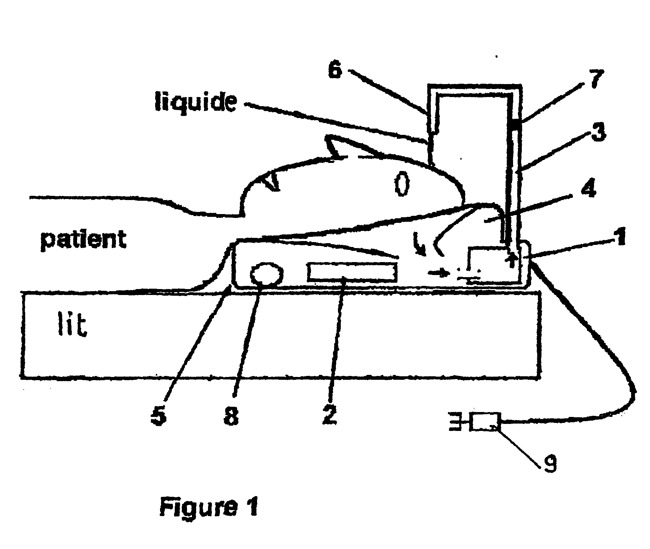

Dans le premier exemple de réalisation représenté à la figure 1 l'appareil décrit est portable, dans ce but tous les éléments sont regroupés.

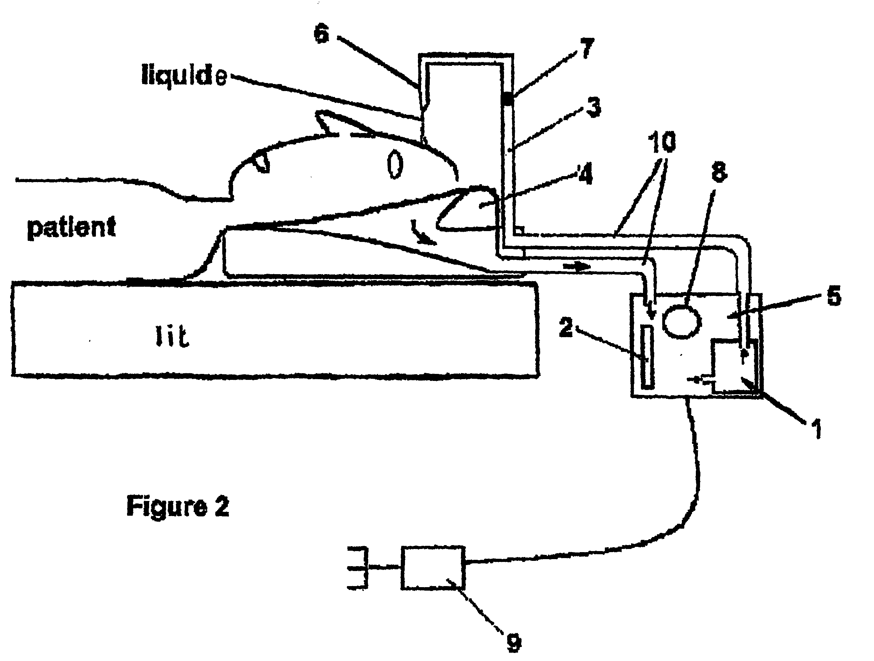

La figure 2 montre une variante où le collecteur est séparé du réservoir contenant le thermostat et la pompe, le réservoir pouvant s'accrocher à un lit et étant relié au collecteur par deux tubes souples 10.

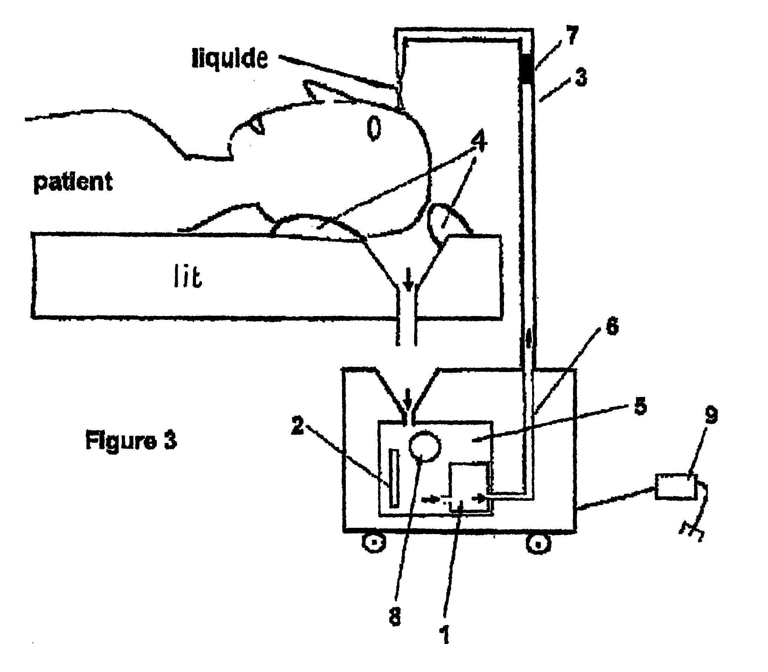

La figure 3 montre une deuxième variante dans laquelle l'appareil n'est pas portable. Le collecteur, le réservoir 5 avec la pompe 1 et le chauffage 2, sont montés sur un chariot à roulettes permettant d'effectuer le Shiorodhara directement sur un patient étendu sur une table de massage.

## Revendications

1. Appareil permettant d'appliquer la technique ayurvédique de Shirodhara, consistant à l'écoulement automatique et en flux continu sur le front d'une personne couchée d'un liquide tempéré induisant un profond état de relaxation physique et mentale,**caractérisé en ce qu'**il comprend un support (4) pour la tête servant de collecteur de récupération dudit liquide, un réservoir (5) pour stocker ledit liquide, une pompe (1) pour délivrer ledit liquide du réservoir à un tube (6) dirigeant le liquide sur le front de la personne, un chauffage à thermostat (2) pour contrôler la température du liquide, une minuterie (8) et statif (3) pour supporter ledit tube (6).

2. Appareil selon la revendication 1, **caractérisé en ce qu'**il comprend un dispositif (7) permettant de déplacer le flux de liquide dans un va-et-vient latéral et continu afin d'opérer un balayage du front.

3. Appareil selon l'une des revendications 1 et 2, **caractérisé en ce que** ledit support (4), ledit collecteur, ledit réservoir (5), ladite pompe (1),ledit tube (6), ledit chauffage à thermostat (2), ladite minuterie (8) et ledit statif (3) constitue un dispositif portable.

## Patentansprüche

1. Gerät zur Anwendung der ayurvedischen Shirodhara-Technik, bestehend aus dem automatischen und kontinuierlichen Ausfluss einer temperierten Flüssigkeit auf die Stirn einer liegenden Person, was zu einer tiefen Entspannung von Körper und Geist führt, **gekennzeichnet dadurch, dass** es eine Kopfunterlage **4**, die als Auffangbehälter für diese Flüssigkeit dient, einen Behälter **5** zur Aufbewahrung dieser Flüssigkeit, eine Pumpe **1** zur Beförderung dieser Flüssigkeit aus dem Behälter zu einem Rohr **6**, welches die Flüssigkeit auf die Stirn der Person weiterleitet, eine Heizung mit Thermostat **2** zur Regelung der Temperatur der Flüssigkeit, einen Timer **8** und ein Stativ **3** zum Halten dieses Rohrs **6** umfasst.

2. Gerät gemäss Anspruch 1, **gekennzeichnet dadurch, dass** es eine Vorrichtung **7** umfasst, welche eine seitliche und kontinuierliche Schwingbewegung der Flüssigkeit ermöglicht, um die Stirn zu überstreichen.

3. Gerät gemäss einen der Ansprüche **1** und **2**, **gekennzeichnet dadurch, dass** diese Kopfunterlage **4,** dieser Auffangbehälter, dieser Behälter **5**, diese Pumpe **1**, dieses Rohr **6**, diese Heizung mit Thermostat 2, dieser Timer **8** und dieses Stativ **3** eine tragbare Vorrichtung darstellen.**P**

## Claims

1. Apparatus allowing the ayurvedic technique of Shirodhara to be used, consisting in inducing a state of deep relaxation by allowing a liquid to automatically fall onto the forehead in a continuous flow, comprising a headrest **4** for maintaining the forehead in a predetermined supine position, a collector for delivering said liquid to a reservoir **5,** a pump **1** for delivering said liquid from said reservoir to a distribution pipe **6** directing fluid toward the forehead, a thermostatic unit **2** for controlling a temperature of the liquid, a timer **8** and a stand **3** supporting the distribution pipe **6**.

2. Apparatus as claimed in claim 1, further comprising a sweeping device **7** for moving the distribution pipe lateral across an area defined by the forehead.

3. Apparatus as claimed in claim **1,** said headrest **4,** said collector, said reservoir **5,** said pump **1,** said distribution pipe **6,** said thermostatic unit **2,** said timer **8** and said stand **3** form a portable device.
